# EUROPEAN PATENT APPLICATION

(11) **EP 4 012 374 A1**
(43) Date of publication of application: **15.06.2022**
(21) Application number: 21210423.6
(22) Date of filing: 25.11.2021
(51) Int. Cl.: G01N 9/26, G01N 33/02, G01N 33/04

(54) **AN ARRANGEMENT FOR CONTINUOUSLY DETERMINING DENSITY OF A FOOD PRODUCT, A METHOD THEREOF**

(30) Priority: 08.12.2020 EP 20212398
(71) Applicant: Tetra Laval Holdings & Finance S.A., 1009 Pully (CH)
(72) Inventor: VIRGILI, Magnus, 247 64 Veberöd (SE); Hast, Martin, 211 52 Malmö (SE)
(74) Representative: Tetra Pak - Patent Attorneys SE

(57) **Abstract**

An arrangement (200) for continuously determining density of a food product in a food handling system (100) comprising a tank. The arrangement (200) comprising: a first pressure sensor (202) for determining a first pressure value of the food product in the tank; a second pressure sensor (204) for determining a second pressure value of the food product in the tank; wherein the first pressure sensor (202) is arranged above the second pressure sensor (204) with a height difference (Δh); a memory (208) holding a specific gravity constant data and a height difference data, wherein the height difference data is related to the height difference (Δh); and a controller (208) configured to determine the density of the food product in the food handling system (100) based on the first pressure value, the second pressure value, the specific gravity constant data and the height difference data.

## Description

### Technical Field

The invention is related to packaging technology. More particularly, it is related to an arrangement for continuously determining density of a food product in a food handling system and a food handling system thereof. It is further related to a method for continuously determining density of the food product and a computer program product for implementing the same.

### Background Art

Today it is common practice to use blank-fed packaging systems for different type of food products, e.g. milk or tomatoes. Examples of such systems are Tetra Rex^{™} and Tetra Recart^{™}, both marketed by Tetra Pak. In short, the blank-fed packaging systems are connected to a food processing line such that the food product can be fed from the food processing line to the blank-fed packaging system. The food processing line may be a line for continuous production, but it may also be a line for batch production.

The blank-fed packaging systems, also referred to as filling machines, come with several advantages. One is that a quick conversion between different volumes for packages both with and without the same bottom format is possible. Another advantage is that it is possible to change between different openings in the food package in an efficient way.

Even though the blank-fed packaging systems come with several advantages, there is nevertheless room for improvement. For instance, unlike a roll-fed packaging machine, e.g. Tetra Brik A3^{™} marketed by Tetra Pak, the blank-fed packaging machines fill each package individually with food product. If having a filling device not being tuned accurately or not being able to be adapted according to different product properties, an effect can be that the packages is not filled consistently. Put differently, the packages may vary in terms of product volume.

A drawback with today's approach is thus that there is a risk that the packages are not filled according to pre-set requirement. To avoid producing packages filled below a volume setpoint, it is today common to have a quality assessment step in which the packages are analyzed to assure that they within pre-set requirements. Since packages not being within the requirements are discarded, an imprecise filling device can result in losses in terms of food product as well as production time. Thus, to be able to provide for more efficient packaging systems, both in terms of financial costs as well as in reduced product losses and production time, more precise filling of packages is requested.

Based on the above, there is a need for improved filling precision for packages, especially within the food industry, such that product losses may be reduced as well as the filling process may be provided in an efficient way without significant cost increases.

### Summary

It is an object of the invention to at least partly overcome one or more of the above-identified limitations of the prior art.

According to a first aspect it is provided an arrangement for continuously determining density of a food product in a food handling system comprising a tank. The arrangement comprising a first pressure sensor for determining a first pressure value of the food product in the tank; a second pressure sensor for determining a second pressure value of the food product in the tank; wherein the first pressure sensor is arranged above the second pressure sensor with a height difference; a memory holding a specific gravity constant data and a height difference data, wherein the height difference data is related to the height difference; and a controller configured to determine the density of the food product in the food handling system based on the first pressure value, the second pressure value, the specific gravity constant data and the height difference data.

Throughout this application, the terms "package(s)" and "food package(s)" are used interchangeably.

The first pressure value may be an absolute pressure value. The second pressure value may be an absolute pressure value.

The height difference may be the distance between the first pressure sensor and the second pressure sensor, wherein the first pressure sensor may be arranged above the second pressure sensor. Thus, the first pressure sensor and the second pressure sensor may be arranged with the distance from each other. The height difference may be calibrated once on installation of the first pressure sensor and the second pressure sensor and should not need to be changed again unless the first pressure sensor and/or the second pressure sensor may change position. The height difference may be stored in the memory during installation of the first pressure sensor and the second pressure sensor. The height difference may be stored as the height difference data in the memory. The first pressure sensor and the second pressure sensor may be mounted on a bar, wherein the first pressure sensor and the second pressure sensor may be arranged on the bar with the height difference. Thus, by mounting the sensors on the bar may provide for that the sensors may be kept in the correct position according to the height difference in a more reliable way. The first pressure sensor and the second pressure may be arranged in a float. Thus, the first pressure sensor and the second pressure sensor may be arranged on different distances from a bottom of the tank.

It should be noted that the height difference may be determined in any way as long as the height difference is a measurable difference. The height difference may be a pre-set height difference.

The specific gravity constant data may be calibrated during installation of the arrangement and should not need to be changed again. Preferably, the specific gravity constant data is 9.82 m/s².

The arrangement is advantageously in that it may provide for a more accurate and efficient food handling process of the food product. By continuously determine the density of the food product may provide for that the density may be a real time density value compared to the approach of today, wherein the density of the food product in the food handling system may be a fixed value. Thus, by continuously determining the density, may provide for that a correct density may be used during operation of the food handling system.

A further advantage with the arrangement is that the arrangement may be arranged in existing food handling system but also during production of the food handling system. Thus, the disclosed arrangement may be used in conventional food handling system as well as in newly produced ones. By having the possibility of introducing the arrangement within already existing food handling system provides for that an improved food handling system may be achieved without any significant cost increases.

A further advantage with the arrangement may be that filling related issues that may occur in the food handling system may be easier to understand as well as easier to avoid. Thus, the food product may be monitored within the food handling system such that the operator may know a real time status of the food product. The real time status of the food product may e.g. relate to the quality of the food product, or the like.

A yet further advantage with the arrangement may be that an improvement in automatization of the food handling system may be achieved because of that the controller may continuously determine the density of the food product. Therefore, a more user-friendly food handling system may be achieved as well.

The controller may further be configured to transmit control data to a filling machine, comprised in the food handling system, based on the density of the food product.

The term "control data" is here meant data formed based on the density and may be used to control settings of the filling machine. The settings that may be controlled based on the control data may be related to opening and closing of a valve on a product pipe such that filing of food product into the package can be initiated by opening the valve and stopped by closing the valve. Thus, by transmitting control data from the controller to the filling machine provides for that the filling machine may be controlled based on the control data. The control data may be a real time control data that may vary based on the density. Thus, an improved control of the filling machine may be achieved with the disclosed arrangement.

The filling machine may be configured to feed the food product into a food package and wherein the controller may be further configured to control a volume of the food product filled in the food package.

The volume may be controlled by the control data transmitted to the filling machine from the controller. The volume may be controlled based on the density of the food product. According to one non-limiting example, if the package may be underfilled or overfilled according to pre-set requirements, the controller may be configured to control the volume that may be filled in the package such that the package may be filled in the correct way, without being underfilled or overfilled. Thus, based on the density, it may be possible to determine the volume of the food product that may be filled in the package.

An advantage by having the controller that may be configured to control the volume of the food product is that a more secure and accurate filling process may be achieved compared to today's approach, wherein operators may determine the volume that may be filled in the food package based on the weight of the food package with the food product and the fixed density of the food product. Thus, with the disclosed arrangement, a continuous filling process may be achieved.

A further advantage by controlling the volume of the food product based on the density may be that the filling process may be more efficient and user-friendly compared to today's approach.

The tank may be a buffer tank placed upstream a filling device of the filling machine.

The buffer tank may balance up and downstream processes such that product losses may be minimized as well as the efficiency may be increased. The buffer tank may be configured to feed additional food product to the filling machine such that the processes may be a continuous process.

By determining the density of the food product in the buffer tank placed upstream the filling machine may increase the efficiency in the filling process. Thus, the operator may be one step ahead such that it may be possible to pre-set the volume that may be filled in the packages based on the density.

An advantage with introducing the arrangement in the filling machine may be that it may be possible to determine if the food product fulfils set requirements before being filled in the food packages.

The tank may be a buffer tank placed in a food processing line placed upstream the filling machine.

The buffer tank may balance up and downstream processes such that product losses may be minimized as well as the efficiency may be increased. The buffer tank may be configured to feed additional food product to the food processing line such that the processes may be a continuous process. The buffer tank may be arranged anywhere in the food processing line. The buffer tank may be arranged where the balancing may be needed.

An advantage with introducing the arrangement in the food processing line may be that it may be possible to determine if the food product fulfils set requirements before being fed to the filling machine.

The arrangement may further comprise a third pressure sensor for determining a third pressure value of the food product, wherein the third pressure sensor is arranged below the second pressure sensor with an additional height difference; wherein the memory holds an additional height difference data, wherein the additional height difference data is related to the additional height difference; and wherein the controller is further configured to determine an additional density of the food product in the food handling system based on the second pressure value, the third pressure value, the specific gravity constant data and the additional height difference data.

It should be noted that the third pressure sensor may be arranged anywhere in the arrangement in relation to the first pressure sensor and/or the second pressure sensor. However, it may be advantageous to arrange the third pressure sensor below the second pressure sensor such that the density of the food product in the bottom of the tank may be determined. Thus, if the additional density may differ from the density, it may indicate that there may be some issues with the food product. According to one non-limiting example, it may indicate that the food product may be sedimented. If that may be the case, it may be possible to install an agitator within the tank holding the food product such that the food product may be mixed. An advantage with this may be that the food product may fulfil the set requirements before being fed to the food package.

Thus, it may be advantageous to install the third pressure sensor in the arrangement such that even more information about the food product may be carried out.

It should be noted that the disclosure may comprise more than three pressure sensors as well.

The arrangement may further comprise a signaling device configured to signaling if the density is outside a pre-set density interval.

The signaling device may be advantageous as it may indicate if the density may be outside the pre-set density interval. This may be advantageous because if the density may be outside the pre-set interval, it may indicate that issues regarding the food product may have occurred. According to one non-limiting example, it may indicate if a bacterial formation may have occurred in the food product or if the food product may have been mixed or produced in a wrong way.

According to a second aspect, it is provided a method for continuously determining density of a food product in a food handling system comprising a tank. The method comprising: determining, by using a first pressure sensor, a first pressure value of the food product; determining, by using a second pressure sensor, a second pressure value of the food product; wherein the first pressure sensor is arranged above the second pressure sensor with a height difference; retrieving, from a memory, a specific gravity constant data and a height difference data, wherein the height difference data is related to the height difference; determining, by using a controller, the density of the food product in the food handling system based on the first pressure value, the second pressure value, the specific gravity constant data and the height difference data.

The method may further comprise transmitting, by using the controller, control settings to a filling machine comprised in the food handling system based on the density of the food product.

The method may further comprise opening a valve and/or a pump in the filling machine such that food product is filled in a food package; and closing the valve and/or the pump in the filling machine based on the density.

The tank may be a buffer tank placed upstream the filling machine.

The method may further comprise: determining, by using a third pressure sensor, a third pressure value of the food product, wherein the third pressure sensor is arranged/placed below the second pressure sensor with an additional height difference; retrieving, from the memory, an additional height difference data, wherein the additional height difference data is related to the additional height difference; and determining, by using a controller, an additional density of the food product in the food handling system based on the first pressure value and/or the second pressure value and the third pressure value, the specific gravity constant data and the additional height difference data.

The may further comprise: transmitting density data to a quality controller arranged downstream the filling machine, wherein the quality controller comprises a scale to weigh the food package with food product; determining weight data of the food package with the food product by using the scale; and determining the volume in the food package based on the density data and the weight data.

Having the quality controller arranged downstream should be construed broadly. For example, the quality controller can be placed as a separate unit downstream the filling machine. However, the quality controller may also be an integral part of the filling machine. For instance, the quality controller may be placed after the product has been filled into the package, but before the package has been folded into its final shape.

This may be advantageous as if the food product for example may comprise different particles, e.g. pulp if the food product is a juice, the density may vary based on the amount of particles in each food package. Thus, the volume may be correct according to pre-set requirements, but the weight may differ or vice versa. Thus, this may provide for a quality control of the food package with the food product. It should be noted that this step may be provided within the filling machine or downstream the filling machine. It should further be noted that this step may be automatically or manually performed.

The method may further comprise signaling, by using a signaling device, if the density may be outside a pre-set density interval.

According to a third aspect, it is provided a food handling system. The food handling system comprising: a food processing line; a filling machine; and an arrangement according to the first aspect.

According to a fourth aspect, it is provided a non-transitory computer-readable storage medium having stored thereon program code portions for implementing the method according to any one of claims 8 to 14 when executed on a device having processing capabilities.

Still other objectives, features, aspects and advantages of the invention will appear from the following detailed description as well as from the drawings.

Effects and features of the second, third and fourth aspects are largely analogous to those described above in connection with the first aspect. Embodiments mentioned in relation to the first aspect are largely compatible with the second, third and fourth aspect. It is further noted that the inventive concepts relate to all possible combinations of features unless explicitly stated otherwise. A further scope of applicability of the present invention will become apparent from the detailed description given below. However, it should be understood that the detailed description and specific examples, while indicating preferred embodiments of the invention, are given by way of illustration only, since various changes and modifications within the scope of the invention will become apparent to those skilled in the art from this detailed description.

Hence, it is to be understood that this invention is not limited to the particular component parts of the device described or steps of the methods described as such device and method may vary. It is also to be understood that the terminology used herein is for purpose of describing particular embodiments only and is not intended to be limiting. It must be noted that, as used in the specification and the appended claim, the articles "a", "an", "the", and "said" are intended to mean that there are one or more of the elements unless the context clearly dictates otherwise. Thus, for example, reference to "a unit" or "the unit" may include several devices, and the like. Furthermore, the words "comprising", "including", "containing" and similar wordings does not exclude other elements or steps.

### Brief Description of the Drawings

Embodiments of the invention will now be described, by way of example, with reference to the accompanying schematic drawings, in which
Fig. 1 illustrates a food handling system.
Fig. 2 illustrates an arrangement for continuously determining density of a food product.
Fig. 3 illustrates an additional arrangement for continuously determining density of a food product.
Fig. 4 is a flow chart illustrating a method for continuously determining density of a food product in the food handling system.

### Detailed description

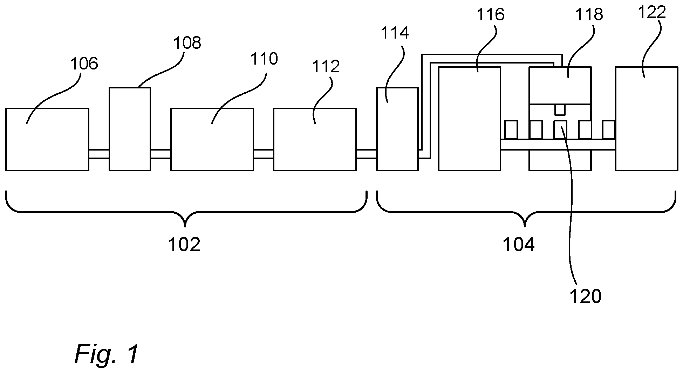

Figure 1 illustrates a food handling system 100 by way of example. The food handling system 100 may comprise a food processing line 102 and a filling machine 104. The food handling system 100 may be configured to process food product by using the food processing line 102. The food handling system 100 may be configured to fill the food product into food packages by using the filling machine 104.

The food processing line 102 may comprise a separator 106 for separating the food product. For example, the separator 106 may be used for separating out nondesired particles from the food product, e.g. dirt that unintentionally ended up in the product.

The food processing line 102 may comprise a buffer tank 108 for holding the food product. By having the buffer tank 108 variations in flow may be compensated for such that a continuous process can be achieved.

The food processing line 102 may comprise a heat exchanger 110 for sterilizing the food product. The food processing line 102 may comprise a homogenizer 112 for homogenizing the food product before being fed to the filling machine 104. In this particular example, the heat exchanger 110 and the homogenizer 112 is placed downstream the buffer tank 108. However, as is readily appreciated by the skilled person other set-ups of the processing line can be used. In addition, the processing line may also comprise several parallel sub-lines such that e.g. production can continue even though parts of the processing line are serviced or cleaned.

The buffer tank 108 may be arranged anywhere in the food processing line 102 where balancing of the food product may be needed. Thus, the buffer tank 108 may be arranged after the separator and/or after the homogenizer. Thus, the food processing line 102 may comprise more than one buffer tank. The filling machine 104 may also, or instead, comprise a buffer tank 114 for holding food product for enabling a steady product flow in the filling machine 104. Put differently, the buffer tank 114 may be configured to provide for that the filling machine can be run continuously.

The filling machine 104 may comprise a ready to fill (RTF) former and sterilization device 116, herein illustrated as one piece of equipment by way of example. The RTF former and sterilization 116 may receive blanks from an external arrangement, e.g. a magazine, wherein the RTF former and sterilization device 116 may form the blanks into food packages 120. The food packages 120 may be so-called ready to fill (RTF) packages, i..e the packages may have one end open such that these may be filled with product and thereafter closed. Instead of using RTF packages, the packages may be bottles without caps applied. Before sealing one of the two ends, and leaving the other end open, the food packages 120 may be sterilized. A top of the food packages may be pre-bent after being sterilized to facilitate the closing of the packages. Even though illustrated that the packages are sterilized in the RTF former and sterilization device 116, other ways of sterilization the packages can be applied. For instance, the packages can be sterilized after being filled and closed, which is the case in e.g. Tetra Recart^{™} systems.

The filling machine 104 may comprise a filler 118 for filling the food product into the food packages 120. The food packages 120 may be filled with a pre-set volume of the food product. The pre-set volume may be based on a pre-set weight of the food package 120 filled with food product as well as a density of the food product. The filling machine 104 may comprise a final former 122 for sealing the other one of the two ends. To be able to meet set requirements in terms of volume if measuring weight of the food product before filling into the packages, having accurate information about the product density provides for increased reliability and in turn that the risk of having too low volume in the packages, i.e. not meeting the set requirements, can be avoided.

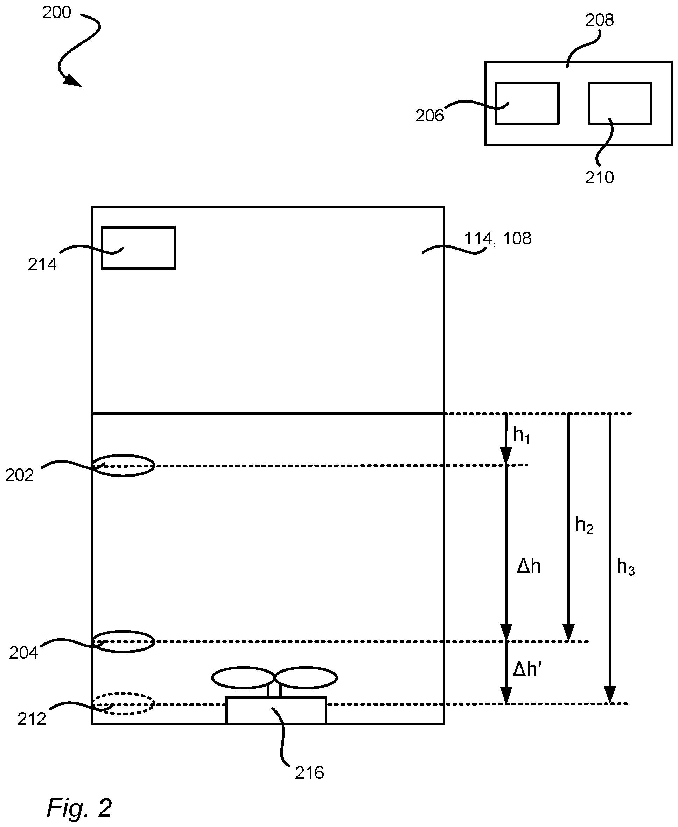

Figure 2 illustrates an arrangement 200 for continuously determining the density of the food product in the food handling system 100 discussed in connection with Fig. 1. The purpose of the arrangement 200 may be to continuously determine the density of the food product such that the food packages 120 may be filled in a correct way, with the correct amount of food product. The arrangement 200 may be arranged in the buffer tank 114 of the filling machine 104. In addition, or alternatively, the arrangement 200 may be arranged in the buffer tank 108 of the of the food processing line 102.

However, it should be noted that the arrangement 200 may be arranged in any tank within the food handling system 100. The arrangement 200 may be used in the food handling system 100 during production of the food handling system 100 such that density measurement can be made continuously during production, i.e. in-line density measurement. The arrangement 200 may be a kit of parts that can be installed in an already existing food handling system 100.

The arrangement 200 may comprise a first pressure sensor 202 and a second pressure sensor 204. The first pressure sensor 202 may be arranged above the second pressure sensor 204. Preferably, both the first pressure sensor 202 and the second pressure sensor 204 may be arranged in the food product. The first pressure sensor 202 may be arranged on a first height h₁. The second pressure sensor 204 may be arranged on a second height h₂. The first sensor 202 may be arranged above the second pressure sensor 204 with a height difference Δh. The height difference Δh may be based on the first height h₁ and the second height h₂. Based on the known sensors of today, the height difference Δh between the first sensor and the second sensor may be in the interval of one to three decimeters. However, it should be noted that the greater height difference Δh, the greater output values, e.g. a first pressure value and a second pressure value. The first pressure sensor 202 may be configured to determine the first pressure value of the food product. The second pressure sensor 204 may be configured to determine the second pressure value of the food product. The first pressure sensor 202 may be configured to determine a level of food product in the tank. The second pressure sensor 204 may be configured to determine a hydrostatic pressure in the bottom of the tank, wherein the hydrostatic pressure relates to the difference between the first pressure value and the second pressure value.

The arrangement 200 may further comprise a controller 208. The controller 208 may comprise a memory 206 and a processor 210. The memory 206 may be configured to hold a specific gravity constant data. The memory 206 may further be configured to hold a height difference data, wherein the height difference data may relate to the height difference Δh. The controller 208 may be configured to continuously determining the density of the food product. The controller 208 may be configured to determining the density based on the first pressure sensor, the second pressure sensor, the specific gravity constant data and the height difference data.

The controller 208 may further be configured to transmit control data to the filling machine 104.The control data may be based on the density of the food product. Thus, the controller 208 may be configured to control the filling machine 104 based on the density.

As discussed above, the filling machine 104 may be configured to feed the food product into the food packages 120. The controller 208 may be configured to control a volume of the food product filled into the food package 120. The volume of food product may be determined based on the density. The controller 208 may be configured to control the volume filled in the food package by controlling one or more valves in the filling machine 104 (not illustrated in the figures). The one or more valves may be configured to be open or closed. If the one or more valves may be open, the food product may be able to be filled in the food package 120. If the one or more valves may be closed, the food product may not be able to be filled in the food package 120. Thus, the controller 208 may be configured to control the one or more valves based on the density, and thereby also control the volume that may be filled in the food package 120 based on the density.

The arrangement 200 may further comprise a signaling device 214. The signaling device 214 may be configured to signal if the density may be outside a pre-set density interval. The signaling device 214 may be configured to signal if the density may be outside the pre-set density interval by using light or sound such that an operator may notice the signaling.

The arrangement 200 may further comprise a third pressure sensor 212. The third pressure sensor 212 may be arranged below the second pressure sensor 204. The third pressure sensor 212 may be arranged on a third height h₃. The third pressure sensor 212 may be arranged in the food product. The third pressure sensor 212 may be arranged below the second pressure sensor 204 with an additional height difference Δh'. The additional height difference Δh' may be based on the second height h₂ and the third height h₃. The third pressure sensor 212 may be configured to determine a third pressure value. However, it should be noted that the third pressure sensor may be arranged anywhere in the arrangement 200. It should further be noted that the additional height difference Δh' may be based on the first height h₁ and the third height h₃. It should be yet further noted that the additional height difference Δh' may be based on the first height hi, the second height h₂ and the third height h₃.

If the arrangement 200 may comprise the third pressure sensor 212, the memory 206 may be configured to hold an additional height difference data, wherein the additional height difference data may relate to the additional height difference Δh'.

If the arrangement 200 may comprise the third pressure sensor 212, the controller 208 may be further configured to determine an additional density of the food product. The additional density of the food product may be based on the second pressure value, the third pressure value, the specific gravity constant data and the additional height difference data. However, it should further be noted that if the additional height difference Δh' may be based on the first height h₁ and the third height h₃, the density may be determined based on the first pressure value, the third pressure value, the specific gravity constant data and the additional height difference data. It should be noted that, although not illustrated, the disclosure may comprise more than three pressure sensors as well.

The additional density may be compared with the density. If the additional density may differ from the density, it may indicate that there may be some issues with the food product. It may indicate that the food product may be sedimented. If that may be the case, it may be possible to install an agitator 216 within the buffer tank 108, 114 holding the food product such that the food product may be mixed. The agitator 216 may be arranged in vicinity to the bottom of the buffer tank 108, 114.

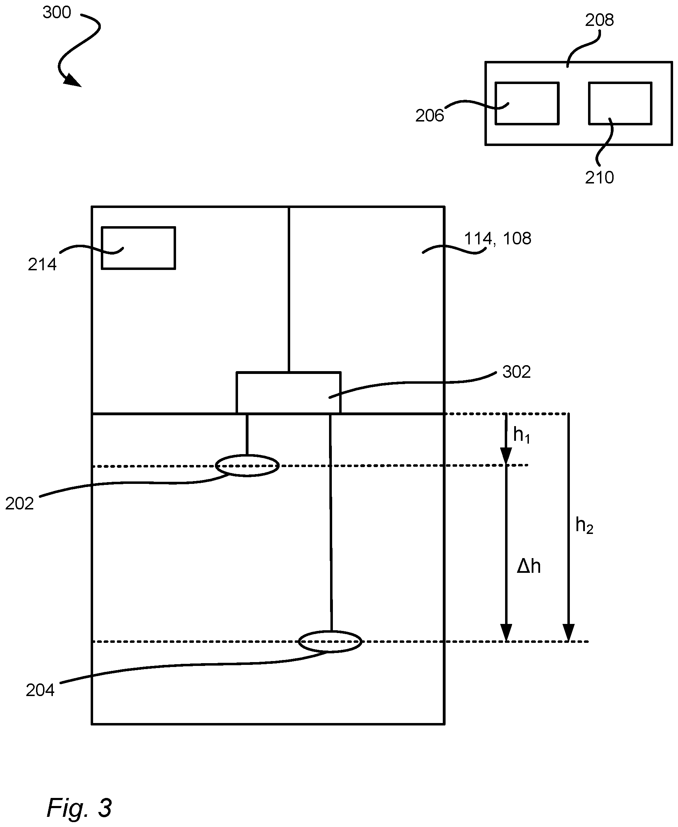

Figure 3 illustrates an additional arrangement 300 for continuously determining the density of the food product in the food handling system 100 discussed in connection with Fig. 1. The additional arrangement 300 may comprise the first pressure sensor 202, the second pressure sensor 204, the buffer tank 108, 114 and the controller 208 as discussed in connection with Fig. 2, and a float 302. In order to avoid undue repetition, reference for the first pressure sensor 202, the second pressure sensor 204, the buffer tank 108, 114 and the controller 208 is made to the above.

The float 302 may be configured to float in the food product. The float 302 may be configured to hold the first pressure sensor 202 and the second pressure sensor 204 such that the sensors 202, 204 are floating in the food product. The sensors 202, 204 may be arranged at different heights in relation to the float 302. This arrangement may be advantageous as it allows for the whole arrangement to be in one such that the whole arrangement may be introduced in the food handling system 100 at once. It should be noted, although not illustrated, the additional arrangement 300 may comprise the third pressure sensor 212 and/or the agitator 216.

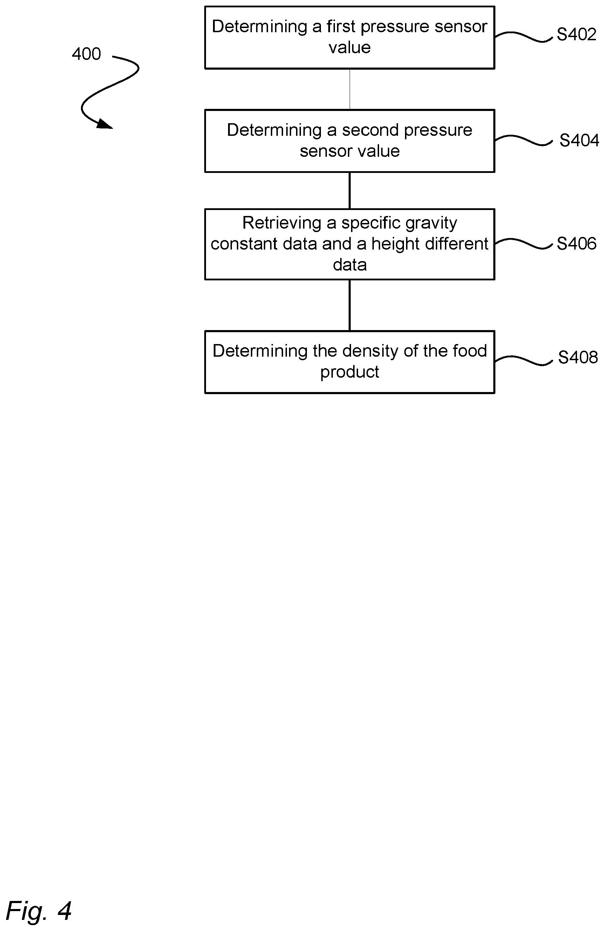

In Fig. 4 it is presented a flow chart illustrating a method 400 for continuously determining density of a food product in a food handling system 100 comprising a tank.

In a first step S402, a first pressure value of the food product may be determined by using a first pressure sensor 202. In a second step S404, a second pressure value of the food product may be determined by using a second pressure sensor 204. The first pressure sensor 202 may be arranged above the second pressure sensor 204 with a height difference Δh.

In a third step S406, a specific gravity constant data and a height difference data may be retrieved from a memory 206. The height difference data may be related to the height difference Δh.

In a fourth step S408, the density of the food product may be determined by using a controller 208. The density of the food product may be based on the first pressure value, the second pressure value, the specific gravity constant data and the height difference data.

Optionally, the method 400 may further comprise transmitting, by using the controller 208, control settings. The control settings may be transmitted to a filling machine 102 comprised in the food handling system 100 based on the density of the food product.

Optionally, the method 400 may further comprise opening a valve in the filling machine 104 such that food product is filled into a food package; and closing the valve in the filling machine 104 based on the density. The valve may be opened and/or closed by using the controller 208. Thus, the controller 208 may be configured to control the valve.

Optionally, the method 400 may further comprise transmit density data to a quality controller arranged downstream the filling machine. The quality controller may comprise a scale to weigh the food package with food product. Determine weight data of the food package with the food product by using the scale and determine the volume in the food package based on the density data and the weight data.

Optionally, the method 400 may further comprise determining, by using a third pressure sensor 212, a third pressure value of the food product. The third pressure sensor 212 may be arranged below the second pressure sensor 204 with an additional height difference. The method 400 may further comprise retrieving, from the memory 206, an additional height difference data, wherein the additional height difference data may be related to the additional height difference. The method 400 may further comprise determining, by using the controller 208, an additional density of the food product in the food handling system 100 based on the first pressure value and/or the second pressure value and the third pressure value, the specific gravity constant data and the additional height difference data.

Optionally, the method 400 may further comprises signaling, by using a signaling device 214, if the density may be outside a pre-set density interval.

The method 400 may be executed on a device having processing capabilities. In order for the method 400 to be executed on the device, a non-transitory computer-readable storage medium may have stored thereon program code portions for implementing the method 400.

From the description above follows that, although various embodiments of the invention have been described and shown, the invention is not restricted thereto, but may also be embodied in other ways within the scope of the subject-matter defined in the following claims.

## Claims

1. An arrangement (200, 300) for continuously determining density of a food product in a food handling system (100) comprising a tank, wherein the arrangement (200) comprising:
a first pressure sensor (202) for determining a first pressure value of the food product in the tank;
a second pressure sensor (204) for determining a second pressure value of the food product in the tank;
wherein the first pressure sensor (202) is arranged above the second pressure sensor (204) with a height difference (Δh);
a memory (206) holding a specific gravity constant data and a height difference data, wherein the height difference data is related to the height difference (Δh); and
a controller (208) configured to determine the density of the food product in the food handling system (100) based on the first pressure value, the second pressure value, the specific gravity constant data and the height difference data.

2. The arrangement (200, 300) according to claim 1, wherein the controller (208) is further configured to transmit control data to a filling machine (104), comprised in the food handling system (100), based on the density of the food product.

3. The arrangement (200, 300) according to claim 2, wherein the filling machine (104) is configured to feed the food product into a food package (120) and wherein the controller (208) is further configured to control a volume of the food product filled in the food package (120).

4. The arrangement (200, 300) according to any one of the preceding claims, wherein the tank is a buffer tank (114) placed upstream a filling device of the filling machine (104).

5. The arrangement (200, 300) according to any one of claims 1-3, wherein the tank is a buffer tank (108) placed in a food processing line (102) placed upstream the filling machine (104).

6. The arrangement (200, 300) according to any one of the preceding claims, further comprises
a third pressure sensor (212) for determining a third pressure value of the food product, wherein the third pressure sensor (212) is arranged below the second pressure sensor (204) with an additional height difference (Δh');
wherein the memory (206) holds an additional height difference data, wherein the additional height difference data is related to the additional height difference (Δh'); and
wherein the controller (208) is further configured to determine an additional density of the food product in the food handling system (100) based on the first pressure value and/or the second pressure value and the third pressure value, the specific gravity constant data and the additional height difference data.

7. The arrangement (200, 300) according to any one of the preceding claims, further comprises a signaling device (214) configured to signaling if the density is outside a pre-set density interval.

8. A method (400) for continuously determining density of a food product in a food handling system (100) comprising a tank, wherein the method (400) comprising:
determining (S402), by using a first pressure sensor (202), a first pressure value of the food product;
determining (S404), by using a second pressure sensor (204), a second pressure value of the food product;
wherein the first pressure sensor (202) is arranged above the second pressure sensor (204) with a height difference (Δh);
retrieving (S406), from a memory (206), a specific gravity constant data and a height difference data, wherein the height difference data is related to the height difference (Δh);
determining (S408), by using a controller (208), the density of the food product in the food handling system (100) based on the first pressure value, the second pressure value, the specific gravity constant data and the height difference data.

9. The method (400) according to claim 8, wherein the method (400) further comprises:
transmitting, by using the controller (208), control settings to a filling machine (104) comprised in the food handling system (100) based on the density of the food product.

10. The method (400) according to any one of the preceding claims, wherein the method (400) further comprises:
opening a valve and/or a pump in the filling machine (104) such that food product is filled in a food package; and
closing the valve and/or the pump in the filling machine (104) based on the density.

11. The method (400) according to any one of the preceding claims, wherein the method (400) further comprises:
transmitting density data to a quality controller arranged downstream the filling machine (104), wherein the quality controller comprises a scale to weigh the food package (120) with food product;
determining weight data of the food package (120) with the food product by using the scale; and
determining the volume in the food package (120) based on the density data and the weight data.

12. The method (400) according to any one of the preceding claims, wherein the method further comprises:
determining, by using a third pressure sensor (212), a third pressure value of the food product, wherein the third pressure sensor (212) is arranged below the second pressure sensor (204) with an additional height difference (Δh');
retrieving, from the memory (206), an additional height difference data, wherein the additional height difference data is related to the additional height difference (Δh'); and
determining, by using a controller (208), an additional density of the food product in the food handling system (100) based on the first pressure value and/or the second pressure value and the third pressure value, the specific gravity constant data and the additional height difference data.

13. The method (400) according to any one of the preceding claims, wherein the method (400) further comprises signaling, by using a signaling device (214), if the density is outside a pre-set density interval.

14. A food handling system (100) comprising:
a food processing line (102);
a filling machine (104); and
an arrangement (200, 300) according to any one of claims 1-7.

15. A non-transitory computer-readable storage medium having stored thereon program code portions for implementing the method according to any one of claims 8 to 14 when executed on a device having processing capabilities.
